Europäisches Patentamt

**European Patent Office**

**Office européen des brevets**

(11) Publication number: **0 093 545**
A2

(12) **EUROPEAN PATENT APPLICATION**

(21) Application number: **83302267.6**

(22) Date of filing: **21.04.83**

(51) Int. Cl.³: **G 01 D 5/34**

---

(30) Priority: **21.04.82 GB 8211460**

(43) Date of publication of application:
**09.11.83 Bulletin 83/45**

(84) Designated Contracting States:
**AT BE CH DE FR GB IT LI LU NL SE**

(71) Applicant: **University of Strathclyde**
**Royal College 204 George Street**
**Glasgow G1 1XW(GB)**

(72) Inventor: **Jackson, John**
**21 Broompark Drive Lesmahagow**
**Glasgow Scotland(GB)**

(72) Inventor: **Singh, Amreek**
**8 Birch Knowe Bishopbriggs**
**Glasgow G64 1TS Scotland(GB)**

(74) Representative: **MacDougall, Donald Carmichael et al,**
**Messrs. Cruikshank & Fairweather 19 Royal Exchange**
**Square**
**Glasgow G1 3AE, Scotland(GB)**

(54) Displacement sensitive transducers.

(57) A displacement sensitive transducer (10) is formed by a body (11) of elastomeric materials such as silicone rubber to which is attached an electro magnetic radiation emitter/ receiver pair (14) or (20,21) arranged such that radiation emitted by the emitter (20) and received by the receiver (21) is attenuated to an extent determined by the radiation path length in the body (11), elastic variations in the geometrical shape of the body (11) arising from displacements of an article (16) being monitored and providing a measure of these displacements.

EP 0 093 545 A2

## DISPLACEMENT SENSITIVE TRANSDUCERS

This invention relates to displacement sensitive transducers.

Known forms of displacement sensitive transducers incorporate an electrically conductive element through which an electrical current is passed and variation in that current arising from variation in the characteristics of the conductive element caused by displacements of an article being monitored give rise to a measure of these displacements.

It is an object of the present invention to provide a displacement sensitive transducer which does not rely upon an electrically conductive element.

According to the present invention there is provided a transducer sensitive to displacements of an article being monitored said transducer comprising electro magnetic radiation emitter/receiver means in combination with an elastomeric body having radiation attenuating properties, the combination being such that radiation emitted by the emitter and received by the receiver of said means is attenuated to an extent determined by the radiation path length in the elastomeric body, elastic variations in the geometrical shape of said body arising from displacements of said article effecting variations in said attenuation and providing a measure of said displacements.

The elastomeric body is preferably a silicone rubber such as EP411. The silicone rubber preferably has no filler so that it is almost transparent but it may incorporate a small amount of filler provided that the filler is not sufficient to render the silicone rubber opaque to the waveband of radiation at which said emitter/receiver means operates.

The emitter/receiver means may operate either in the visible waveband or in the infra red waveband of the electro magnetic spectrum. In one form the emitter/receiver means comprises a diode and phototransistor pair secured to the elastomeric body.

. . Embodiments of the present invention will now be described by way of example with reference to the accompanying drawings in which:

Fig. 1 illustrates a first form of transducer according to the present invention;

Fig. 2 illustrates a second form of transducer according to the present invention; and

Fig. 3 illustrates a displacement monitoring system incorporating a transducer according to the present invention.

In the first embodiment the transducer 10 comprises an elastomeric body 11 having front and rear faces 12A, 12B respectively. A reflector 13 is located on face 12B and an emitter/receiver pair 14 is located on the front face 12A. The transducer 10 is also provided with an adhesive layer 15 extending between faces 12A and 12B whereby the transducer may be attached to an article 16 the displacements of which are to be monitored. In this arrangement the emitter/receiver pair 14 is arranged so that the emitter emits radiation in the direction of the reflector 13 and radiation reflected thereby is received by the receiver of the pair 14 so that the radiation path length in the elastomeric body 11 is twice the distance between faces 12A and 12B. Displacements in the article 16 either by way of extension or contraction of article 16 in the plane of the adhesive layer 15 result in variation of the radiation path length within the body 11. Also displacements of the article 16 perpendicular to the plane of the adhesive layer 15 result in distortion of the body 11 which also causes variation of the radiation path length therein. In each case the elastic variations in the geometrical shape of the body 11 arising from displacements of the article 16 effect variations in the attenuation to which the radiation emitted by the emitter of the pair 14 is subjected because of the radiation attenuating properties of the body 11 and these variations provide a measure of the displacements of the article 16.

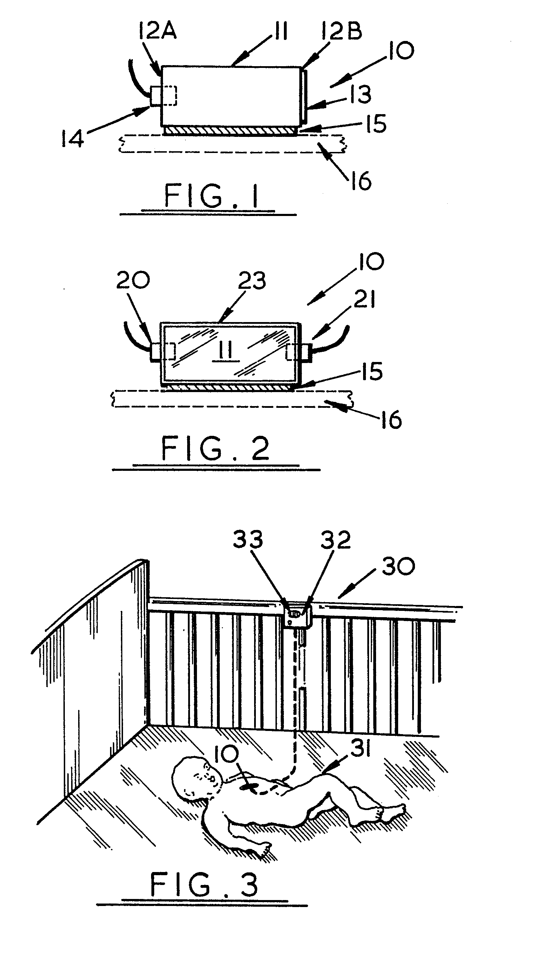

In the embodiment of Fig. 2 only the radiation emitter 20 is located at face 12A of the body 11 whilst the radiation receiver 21 is located at the face 12B of body 11 and there is no reflector 13.

In either of the embodiments the emitter/receiver pair 14 or 20,21 may operate in the visible waveband of the electro magnetic spectrum. In this case it is preferred that the body 11 is provided with an opaque coating 23 (Fig. 2) in order to isolate the receiver 21 from ambient electro magnetic radiation. The radiation emitter pair 14 alternatively may operate in the ultra violet or infra red wavebands of the electro magnetic spectrum in which case it may be possible to dispense with the coating 23.

The body 11 is preferably a room temperature vulcanised silicone rubber such as EP411 either with no filler or with only a small amount of filler. The coating 23 may be a silicone rubber such as C2005 filled with calcium or other opaque filler. The radiation emitter 20 may be a light emitting diode such as that marketed by Radio Spares under their stock number 306-077 which operates in the infra red waveband in which case the receiver 21 is preferably the Radio Spares phototransistor, stock number 306-083 which also operates in the infra red waveband.

In the embodiments of Figs. 1 and 2 the emitter/ receiver pair 14 or 20,21 is directly attached to the elastomeric body 11 preferably by moulding the body 11 to the pair 14 or 20,21. However if so desired the pair 14 or 20,21 may be remote from the body 11 and radiation guided from and to the pair 14 or 20,21 by means of optical fibres.

As is illustrated in Fig. 3 the transducer 10 may be used as part of a system 30 for monitoring the respiration of an infantile patient 31 so that the transducer 10 provides an output signal the attenuation of which varies regularly consequential to the regular breathing of the patient 31 and signal processing circuitry 32 is effective to operate an alarm device 33 in the event of the absence of attenuation variation in the output signal.

## CLAIMS

1. A transducer sensitive to displacements of an article being monitored characterised in that said transducer (10) comprises electro magnetic radiation emitter/receiver means (14) in combination with an elastomeric body (11) having radiation attenuating properties, the combination being such that radiation emitted by the emitter (20) and received by the receiver (21) of said means (14) is attenuated to an extent determined by the radiation path length in the elastomeric body (11), elastic variations in the geometrical shape of said body (11) arising from displacements of said article (16) effecting variations in said attenuation and providing a measure of said displacements.

2. A transducer as claimed in claim 1, characterised in that said elastomeric body (11) is externally provided with an opaque coating (23) in order to isolate said receiver (21) from ambient electro magnetic radiation.

3. A transducer as claimed in claim 1 or claim 2, characterised in that said elastomeric body (11) comprises silicone rubber.

4. A transducer as claimed in any preceding claim, characterised in that said emitter/receiver means (14) operates in the infra red waveband of the electro magnetic spectrum.

5. A transducer as claimed in any one of claims 1-3, characterised in that said emitter/receiver means (14) operates in the visible waveband of the electro magnetic spectrum.

FIG. 1

FIG. 2

FIG. 3